# FASCICULE DE BREVET EUROPEEN

(11) **EP 0 708 108 B1**
(45) Date de publication et mention de la délivrance du brevet: **12.11.1997**
(21) Numéro de dépôt: 95402038.4
(22) Date de dépôt: 08.09.1995
(51) Int. Cl.: C07F 7/08, C07D 249/20, C07F 7/21, A61K 7/42, A61K 7/00

(54) **Nouveaux filtres solaires, compositions cosmétiques photoprotectrices les contenant et utilisations**
Sonnenfilter, diese enthaltende kosmetische Lichtschutzzusammensetzungen sowie deren Verwendung
Solar filtres, cosmetic sunscreen compositions containing them, and their application

(30) Priorité: 07.10.1994 FR 9412004
(43) Date de publication de la demande: 24.04.1996
(73) Titulaire: L'OREAL, 75008 Paris (FR)
(72) Inventeur: Richard, Hervé, F-93420 Villepinte (FR); Leduc, Madeleine, Rue des Boulets, F-75011 Paris (FR); Lagrange, Alain, F-77700 Couvray (FR)
(74) Mandataire: Andral, Christophe André Louis

(56) Documents cités:
- EP-A- 0 388 218
- EP-A- 0 392 883
- WO-A-93/04665
- WO-A-93/10745
- WO-A-94/06404
- WO-A-94/10588
- US-A- 5 164 462

## Description

La présente invention concerne de nouveaux composés du type diorganosiloxanes, à chaines courtes, linéaires ou cycliques, ou du type triorganosilanes, présentant pour caractéristique commune d'avoir tous au moins une fonction alcoxybenzotriazole, ces composés étant plus particulièrement utilisables à titre de filtres organiques solaires dans des compositions cosmétiques destinées à la protection de la peau et des cheveux contre le rayonnement ultraviolet. L'invention concerne également l'utilisation desdits composés dans l'application cosmétique susmentionnée, ainsi que les compositions cosmétiques à propriétés améliorées les contenant. Elle concerne enfin également, à titre de produits industriels nouveaux et utiles en soi, notamment comme filtres pour compositions cosmétiques photoprotectrices de la peau et des cheveux, les alcoxybenzotriazoles à insaturation éthylénique utilisés dans la synthèse des nouveaux composés mentionnés ci-dessus.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreux composés destinées à la photoprotection (UV-A et/ou UV-B) de la peau ont été proposées à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber selectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence).

Parmi tous les composés aromatiques qui ont été préconisés à cet effet, on peut citer notamment les dérivés de l'acide p- aminobenzoïque, les dérivés du benzylidènecamphre, les dérivés de l'acide cinnamique et les dérivés du benzotriazole. Cependant, certaines de ces substances ne présentent pas toutes les propriétés requises pour une utilisation convenable comme filtres UV dans les compositions antisolaires. En particulier, leur pouvoir filtrant intrinsèque peut être insuffisant, leur solubilité dans les différents type de formulations utilisés en matière de protection solaire n'est pas toujours suffisamment bonne (liposolubilité notamment), elles peuvent ne pas posséder une stabilité suffisante à la lumière (photostabilité) et elles peuvent également présenter une mauvaise résistance à l'eau et à la sueur. Il est également souhaitable que ces substances filtrantes ne pénètrent pas dans la peau.

Le brevet WO 94/06404 au nom de la Demanderesse décrit des organosiloxanes à fonction benzotriazole à titre de filtre du rayonnement UV.

Ainsi, dans le cas plus particulier des substances filtres de type benzotriazole, on a cherché à obtenir des produits à propriétés améliorées, en particulier au niveau de leur liposolubilité et de leur caractère cosmétique, en venant fixer par greffage (hydrosilylation) le groupement filtrant benzotriazole sur une chaine macromoléculaire de type silicone (organopolysiloxane). Cette technique, décrite dans la demande de brevet EP 0 392 883 au nom de la Demanderesse, conduit certes à des composés intéressants (ces produits sont connus sous le nom général de "silicones filtres"), mais le caractère liposoluble de ces derniers peut encore apparaitre comme insuffisant et, de plus, afin d'obtenir des propriétés filtrantes satisfaisantes avec ce type de produits, il est souvent nécessaire de mettre en oeuvre des quantités relativement importantes de ces polymères filtres, ce qui se traduit par de mauvaises propriétés cosmétiques au niveau des formulations qui les contiennent.

La présente invention vise à résoudre les problèmes ci-dessus en proposant de nouveaux composés, de type silicones filtres, à fonction benzotriazole, qui présentent des propriétés améliorées, notamment au niveau de leur solubilité dans les corps gras, de leurs propriétés cosmétiques, et de leur pouvoir d'absorption intrinsèque vis à vis des UV.

Plus précisemment encore, il a été trouvé, selon la présente invention, qu'en associant, notamment par réaction d'hydrosylilation, un ou plusieurs dérivés de benzotriazole particuliers, à savoir plus présisément des alcoxybenzotriazoles, avec une chaine silicone particulière, linéaire ou cyclique, ou un silane particulier, il était possible d'aboutir à de nouveaux composés du type silicones filtres obviant aux inconvénients des silicones filtres de l'art antérieur, ces nouveaux composés présentant en particulier des propriétés filtrantes très élevées, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet.

La présente invention a ainsi pour premier objet de nouveaux composés qui sont caractérisés par le fait qu'ils répondent à l'une des formules (1) à (3) suivantes : 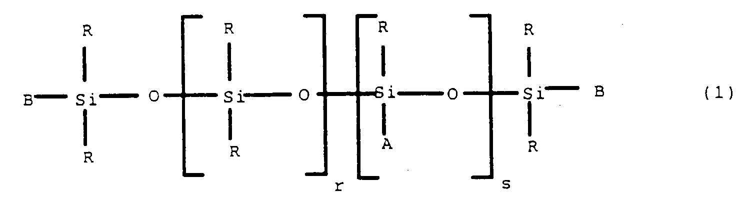 ou 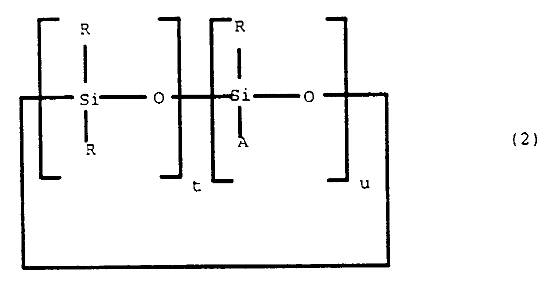 ou

A-Si(R')₃ (3)

formules (1) à (3) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en C₁-C₈, ou phényle,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (4) suivante : 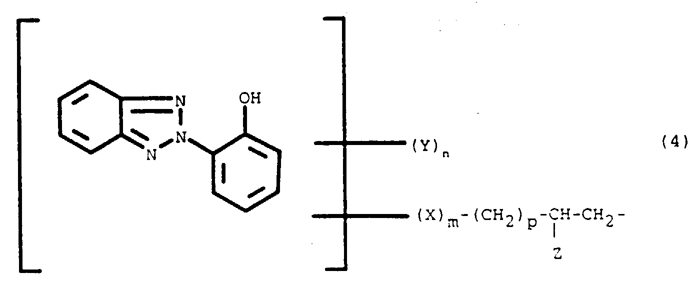 formule (4) dans laquelle :
   - Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
   - X représente O ou NH,
   - Z représente l'hydrogène ou un radical alkyle en C₁-C₄,
   - n est un nombre entier compris entre 0 et 3 inclusivement,
   - m est 0 ou 1, étant entendu que lorsque m est nul, ou lorsque m est égal à 1 et que X représente NH, alors nécéssairement (i) n ne peut être nul et (ii) l'un au moins des Y est un radical alcoxy,
   - p représente un nombre entier compris entre 1 et 10, inclusivement.

Dans les formules (1) à (3) ci-dessus, A représente donc le groupement dérivé du benzotriazole qui, après fixation sur la chaine courte siliconée de départ ou sur le silane de départ, confère aux composés de type diorganosiloxanes linéaires (formule (1)) ou cycliques (formule (2)) ou de type triorganosilanes (formule (3)) des propriétés absorbantes vis à vis de l'ultraviolet dans une zone de longueur d'onde pouvant aller de 280 à 400 nm. Comme indiqué précédemment, et comme cela ressort de la définition de la formule (4) donnée ci-dessus, ce groupement présente nécessairement au moins une fonction alcoxy fixée sur l'un au moins des deux noyaux aromatiques du benzotriazole, cette fonction alcoxy pouvant être apportée soit par un substituant Y soit par le chainon qui assure l'accrochage du benzotriazole à la chaine silicone ou au silane. L'un des grands avantages des composés selon l'invention est que l'on peut obtenir, selon la position occupée par ce ou ces groupements alcoxy sur le motif filtrant A, des filtres soit purement UV-A ou au contraire purement UV-B, et ceci avec des coefficients d'extinction dans tous les cas bien supérieurs à ceux obtenus avec les silicones filtres de l'art antérieur. Ainsi, à titre purement d'exemple, les dérivés possédant un groupement alcoxy en position ortho du groupement hydroxy porté par le noyau benzénique (position 3) donnent des silicones filtres absorbant majoritairement dans l'UV-B, alors que les dérivés possédant un groupement alcoxy en position méta (i.e. positions 4 et/ou 6) de ce même groupement hydroxy donnent des silicones filtres absorbant majoritairement dans l'UV-A.

Comme cela ressort de la formule (4) donnée ci-dessus, l'accrochage du chainon -(X)ₘ-(CH₂)ₚ-CH(Z)-CH₂- sur le motif benzotriazole, qui assure donc le raccordement dudit motif benzotriazole à l'atome de silicium de la chaine siliconée ou du silane, peut, selon la présente invention, se faire dans toutes les positions disponibles offertes par les deux noyaux aromatiques du benzotriazole :

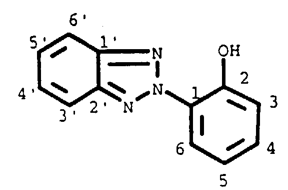

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5.

De même, l'accrochage du motif subsituant Y peut se faire dans toutes les autres positions disponibles au sein du benzotriazole. Toutefois, de préférence, cet accrochage se fait en position 3, 4, 4', 5 et/ou 6.

Dans les formules (1) à (3) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R, R' et B préférés selon l'invention sont les radicaux méthyle, éthyle, propyle, n-butyle, n-octyle et éthyl-2 hexyle. Encore plus préférentiellement, les radicaux R, R' et B sont tous des radicaux méthyle.

Parmi les composés de formules (1) à (3) ci-dessus, on préfère mettre en oeuvre ceux répondant à la formule (1) ou à la formule (2), c'est à dire des diorganosiloxanes à chaine courte, linéaire ou cyclique.

Parmi les diorganosiloxanes linéaires ou cycliques rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R est alkyle et encore plus préférentiellement est méthyle,
- B est alkyle et encore plus préférentiellement méthyle (cas des composés linéaires de formule (1)),
- r est compris entre 0 et 3 inclusivement ; s est compris entre 0 et 3 inclusivement (cas des composés linéaires de formule (1)),
- t+u est compris entre 3 et 5 (cas des composés cycliques de formule (2)),
- n est non nul, et de préférence égal à 1 ou 2, et Y est alors choisi parmi méthyle, ter.-butyle ou alcoxy en C₁-C₄, mais de préférence parmi alcoxy en C1-C4, et encore plus préférentiellement est méthoxy,
- Z est hydrogène ou méthyle,
- X représente O (m ≠ 0),
- p est égal à 1,
étant donc entendu que, dans tous les cas, au moins une fonction alcoxy est directement accrochée sur le dérivé de benzotriazole.

Pour préparer les filtres siliconés de formule (1) et (2), on peut procéder classiquement en mettant en oeuvre une réaction d'hydrosylilation (à savoir 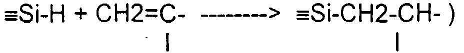 à partir de la silicone correspondante dans laquelle, par exemple, tous les radicaux A sont des atomes d'hydrogène. Cette silicone de départ est dénommée par la suite dérivé à SiH ; les groupes SiH peuvent être présents dans la chaîne et/ou aux extrémités de la chaîne silicone. Ces dérivés à SiH sont des produits bien connus dans l'industrie des silicones et sont généralement disponibles dans le commerce. Ils sont par exemple décrits dans les brevets américains US-A- 3 220 972, US-A- 3 697 473 et US-A- 4 340 709.

Ce dérivé à SiH peut être donc représenté soit par la formule (1 bis) suivante : 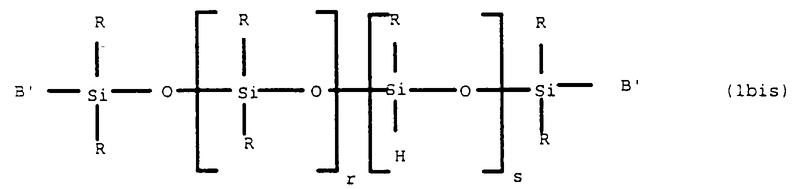 dans laquelle R, r et s ont la signification donnée ci-dessus pour la formule (1) et les radicaux B', identiques ou différents, sont choisis parmi les radicaux R et un atome d'hydrogène,
soit par la formule (2bis) suivante : 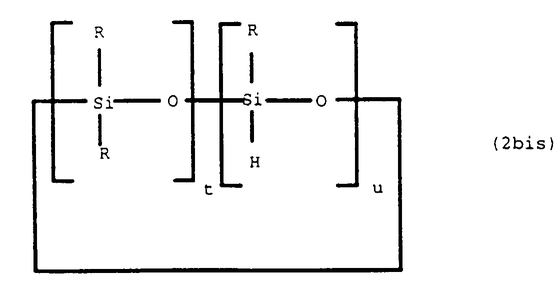 dans laquelle R, t et u ont la signification donnée ci-dessus pour la formule (2).

Sur ce dérivé à SiH de formules (1bis) ou (2bis), on effectue donc une réaction d'hydrosilylation classique, opérée en présence d'une quantité catalytiquement efficace d'un catalyseur au platine, sur un dérivé organique de benzotriazole de formule (4bis) suivante : 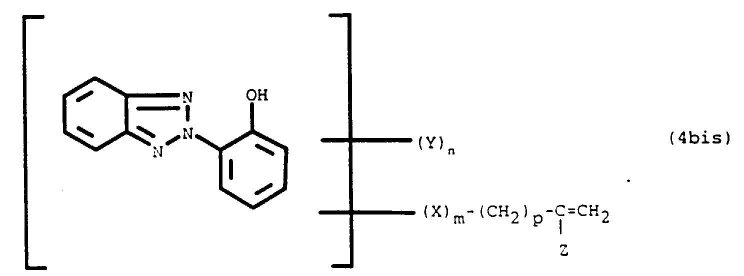 dans laquelle Y, X, Z, n, m et p ont la signification donnée ci-dessus pour la formule (4).

Des procédés convenant à la préparation des produits de formule (4bis) ci-dessus sont notamment décrits dans les brevets US- 4 316 033 et US- 4 328 346.

En outre, les détails des conditions opératoires à suivre pour conduire la réaction d'hydrosylilation entre les composés de formule (1bis) ou (2bis) ci-dessus avec le composé de formule (4bis) ci-dessus sont donnés dans la demande de brevet EP- 0 392 883 précitée, dont l'enseignement est, à cet égard, totalement inclus à titre de référence dans la présente description.

Concernant la préparation des filtres de type triorganosilanes de formule (3) donnée précédemment, on peut procéder comme indiqué ci-dessus, toujours par réaction d'hydrosylilation, entre un silane de départ de formule (R')₃Si-H (formule (3bis), dans laquelle R' a la même signification que pour le composé de formule (3), et un dérivé organique de benzotriazole de formule (4bis) définie ci-avant.

Comme indiqué précédemment, les dérivés organiques de benzotriazole à insaturation monoéthylénique de formule (4bis) ci-dessus constituent des produits nouveaux et utiles en soi, et à ce titre, font également partie intégrante de la présente invention, de même que leur utilisation comme filtres solaires dans le domaine des UV-A et/ou UV-B, en particulier dans des compositions cosmétiques destinées à la photoprotection de la peau ou des cheveux.

Par rapport aux silicones filtres de l'art antérieur telles que décrites dans la demande de brevet EP 0 392 883, les silicones filtres selon l'invention présentent donc une ou plusieurs différences structurelles essentielles, à l'origine de leurs propriétés remarquables : les chaines siliconées, sur lesquelles sont greffés le motif benzotriazole, sont tout d'abord beaucoup plus courtes ; ensuite, le motif dérivé du benzotriazole porte toujours au moins une fonction alcoxy.

Comme indiqué précédemment, les composés de formules (1) à (3) ci-dessus présentent un excellent pouvoir filtrant intrinsèque à l'égard du rayonnement ultraviolet (UV-A ou UV-B, selon la structure du produit). En mélangeant des produits de structure différente, c'est à dire plus précisément en mélangeant des produits conformes à l'invention présentant une activité purement UV-A avec des produits conformes à l'invention présentant une activité purement UV-B, il est ainsi possible de disposer d'une composition qui présentera globalement une activité filtrante exceptionnelle dans toute la gamme des UV nocifs (UV-A + UV-B), ce qui constitue un avantage considérable. En outre, de par leur caractère fortement liposoluble, les composés de formule (1) à (3) ci-dessus peuvent être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. Enfin, leurs propriétés cosmétiques sont très bonnes, à savoir en particulier que ces produits, par rapport aux silicones filtres de l'art antérieur, sont moins collantes et apportent plus de douceur.

La présente invention a donc aussi pour objet une composition cosmétique comprenant, dans un support cosmétiquement acceptable contenant de préférence au moins une phase grasse ou un solvant organique, une quantité efficace d'au moins un composé de formules (1) à (3) définies ci-avant.

Les composés de formules (1) à (3) sont généralement présents dans des proportions comprises entre 0,1 % et 20 % en poids, de préférence entre 0,5 % et 10 % en poids, par rapport au poids total de la composition.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et eventuellement être conditionnée en aérosol et se présenter sous forme de mousse ou de spray.

Elle peut contenir les adjuvants cosmétiques habituellement utilisés dans le domaine, tels que des corps gras, des solvants organiques, des silicones, des épaississants, des adoucissants, des filtres solaires complémentaires, des agents anti-mousses, des agents hydratants, des parfums, des conservateurs, des tensio-actifs, des charges, des sequestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou par une cire ou leurs mélanges, des acides gras, des esters d'acides gras, des alcools gras, la vaseline, la paraffine, la lanoline, la lanolinehydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a aussi pour objet un procédé de protection de la peau et cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1), (2) ou (3), ou bien encore d'un dérivé de benzotriazole de formule (4bis), tels que définis ci-avant.

Les exemples qui suivent illustrent l'invention sans toutefois en limiter la portée.

### EXEMPLE 1

Cet exemple illuste la préparation d'un composé conforme à l'invention de formule: 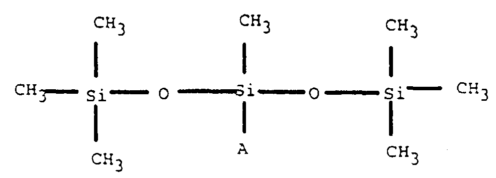 dans laquelle A représente : 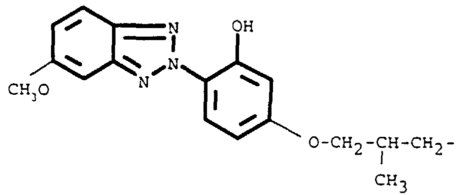 (ce produit correspond à un composé de formule (1) pour lequel R = B = CH₃ ; r = 0 ; s = 1 ; Y = OCH₃ en position 4' ; n = 1 ; m = 1 et X = O ; p = 1 ; Z = CH₃).

### a) Première étape : préparation du 4-(4-méthoxy-2-nitro-phénylazo)-benzène-1,3-diol

Dans un réacteur contenant 44 g (soit 0,4 mole) de résorcinol dissout dans un mélange de 400 ml d'éthanol et 400 ml d'eau, on ajoute, à une température comprise entre 5 et 10°C et en une heure, une solution de sel de diazonium préalablement obtenue par diazotation de 67,2 g (soit 0,4 mole) de 4-méthoxy-2-nitro aniline contenu dans 150 ml d'HCl concentré par une solution de nitrite de sodium à 28 g (soit 0,4 mole) dans 100 ml d'eau à 0-5°C. On laisse sous agitation pendant 2 heures. Le précipité rouge est filtré puis rincé abondamment à l'eau. On obtient ainsi 86 g (rendement: 74 %) du produit désiré.

### b) Deuxième étape : préparation du 2-(4-méthoxy-2-nitro-phénylazo)-5-(2-méthyl-allyloxy)-phénol

Dans un ballon tout équipé, on introduit 81 g (soit 0,28 mole) du produit obtenu précédemment, 220 ml de DMF et 42,6 g (soit 0,308 mole) de carbonate de potassium. On porte le mélange à 80-90°C puis on y introduit goutte à goutte et en 30 minutes 28 g (soit 0,308 mole) de chlorure de méthallyle. Puis on laisse le tout à 90°C sous agitation pendant 4H30. On verse ensuite le mélange réactionnel dans 200 g d'eau glacée et on filtre le précipité obtenu. Ce précipité est ensuite repris dans du dichlorométhane, séché sur sulfate de sodium, concentré et passé sur lit de Silice 60. On obtient, après évaporation du solvant et séchage, 47 g d'une poudre rouge (rendement : 49 %) constituée du produit désiré.

### c) Troisième étape : préparation du 2-(5-méthoxy-benzotriazol-2-yl)-5-(2-méthylallyloxy)-phénol

Dans un ballon, on place 32,54 g (soit 0,134 mole) du produit obtenu précédemment et 400 ml d'éthanol. On y ajoute par portions et en une demi-heure 48,4 g (soit 0,269 mole) de glucose en solution dans 400 ml de soude 2N, et on laisse le tout sous agitation pendant une nuit. On ajoute ensuite 44,4 g de zinc en poudre, et on laisse le tout sous agitation pendant 3 heures. Le mélange est ensuite filtré, le résidu est lavé au dichlorométhane. Le filtrat est extrait au dichlorométhane. Les jus organiques sont rassemblés, lavés à l'eau et séchés. Après concentration et séchage, on obtient 36,6 g d'une poudre de couleur marron-gris. On la recristallise dans de l'éthanol pour obtenir ainsi 41,8 g (rendement : 65%) du produit désiré de formule :

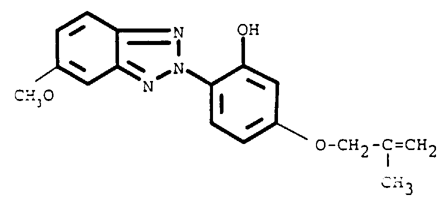

### d) Quatrième étape : préparation du produit final désiré

Dans un réacteur tout équipé, on charge 21,8 g du produit obtenu précédemment et 35 ml de toluène. Le mélange est porté à 80°C sous azote. On ajoute le catalyseur (complexe à 3-3,5% poids de Pt dans du cyclovinylméthylsiloxane de Hüls Petrarch PC085 : 100 µl), puis on ajoute goutte à goutte et en 5 minutes 16,5 g d'heptaméthyltrisiloxane. Le mélange hétérogène est porté au reflux pendant 8 heures sous azote. On concentre le milieu réactionnel et on effectue ensuite une chromatographie sur silice sous pression (éluant : heptane avec un gradient de CH2Cl2). On récupère alors 25 g d'un solide que l'on recristallise dans du méthanol pour obtenir finalement 23,5 g (rendement : 63 %) du produit final désiré.

Les caractéristiques en absorption UV (mesurées dans l'éthanol) de ce produit sont les suivantes :
λₘₐₓ : 349 nm εₘₐₓ : 27 200
Ce produit peut donc être utilisé très efficacement à titre de filtre solaire actif dans l'UV-A.

### EXEMPLE 2

En suivant le même mode opératoire que celui donné à l'exemple 1 (à cette seule différence près que, à l'étape 1, la 4-méthoxy-2- nitro aniline a été ici remplacé par de la 2-nitro aniline), on a préparé 8,5 g (rendement : 67 %) d'un autre composé conforme à l'invention et répondant à la formule suivante : 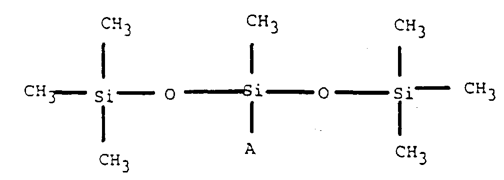 dans laquelle A représente cette fois :

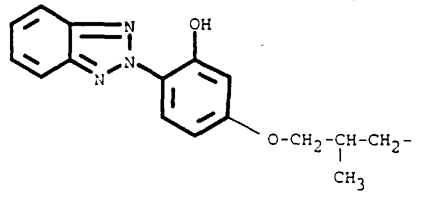

Ce produit ne diffère donc de celui de l'exemple 1 précédent que par l'absence sur le motif benzotriazole du substituant OCH₃.

Les caractéristiques en absorption UV (mesurées dans l'éthanol) de ce produit sont les suivantes :
λₘₐₓ : 342 nm εₘₐₓ : 24 500
Ce produit peut donc être utilisé très efficacement à titre de filtre solaire actif dans l'UV-A.

On notera que le produit intermédiaire obtenu à l'issue de l'étape n°3 dans la chaine du procédé avait pour formule :

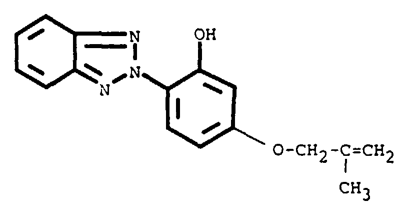

### EXEMPLE 3

En suivant le même mode opératoire que celui donné à l'exemple 1 (à cette seule différence près que, à l'étape 1, le résorcinol a été ici remplacé par le 4-méthyl-2-(2-méthyl-allyloxy)-phénol), on a préparé 2 g (rendement : 54%) d'un autre composé conforme à l'invention et répondant à la formule suivante : 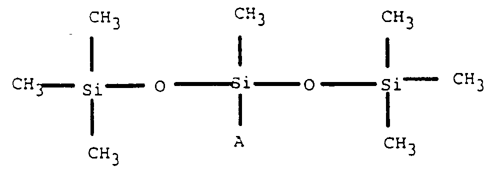 dans laquelle A représente cette fois :

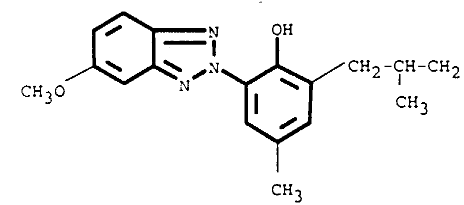

Les caractéristiques en absorption UV (mesurées dans l'éthanol) de ce produit sont les suivantes :
λₘₐₓ : 303 nm εₘₐₓ : 10 900
λₘₐₓ : 348 nm εₘₐₓ : 22 200
Ce produit peut donc être utilisé très efficacement à titre de filtre solaire moyennement actif dans l'UV-B et fortement actif dans l'UV-A.

On notera que le produit intermédiaire obtenu à l'issue de l'étape n°3 dans la chaine du procédé avait pour formule :

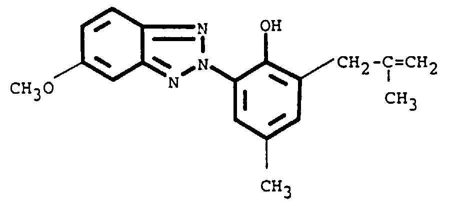

### EXEMPLE 4

En suivant le même mode opératoire que celui donné à l'exemple 1 (à ces différences près que, d'une part, à l'étape 1, la 4-méthoxy-2 nitro aniline et le résorcinol ont été ici remplacés respectivement par de la 2-nitro aniline et de l'eugénol et que, d'autre part, l'étape n°2 a été supprimée), on a préparé 13 g (rendement : 67 %) d'un autre composé conforme à l'invention et répondant à la formule suivante : 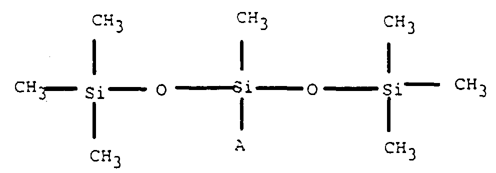 dans laquelle A représente cette fois :

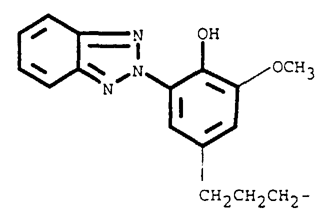

Les caractéristiques en absorption UV (mesurées dans l'éthanol) de ce produit sont les suivantes :
λₘₐₓ : 310 nm εₘₐₓ : 17 800
Ce produit peut donc être utilisé très efficacement à titre de filtre solaire actif dans l'UV-B.

On notera que le produit intermédiaire obtenu avant mise en oeuvre de la dernière étape dans la chaine du procédé avait pour formule :

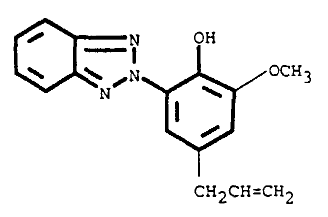

### EXEMPLE 5

On illustre ici une formulation concrète de composition cosmétique antisolaire conforme à l'invention, à savoir une crème antisolaire.
- Composé de l'exemple 1 5 g
- Mélange d'alcool cétylstéarylique et d'alcool cétylstéarylique oxyéthyléné à 33 moles d'OE ("SINNOVAX AO" de chez HENKEL) 7 g
- Mélange de mono et distéarate de glycérol non autoémulsifiable 2 g
- Alcool cétylique 1,5 g
- Benzoate d'alcools en C₁₂-C₁₅ ("FINSOLV TN" de chez WITCO) 20 g
- Polydiméthylsiloxane 1,5 g
- Glycérine 17,5 g
- Parfum, conservateur qs
- Eau qsp 100 g

Cette crème est préparée selon les techniques classiques de préparation d'émulsions en dissolvant le filtre dans la phase grasse contenant les émuisionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40 °C on ajoute enfin parfum et conservateur.

## Revendications

1. Composés de formules : 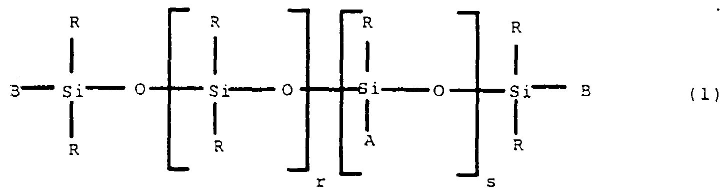 ou 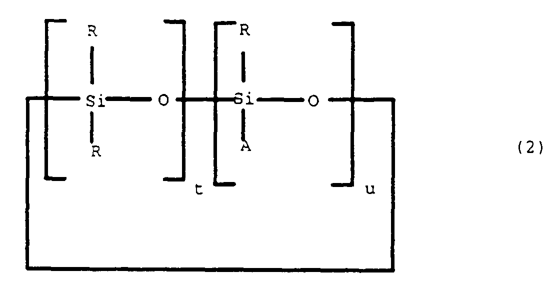 ou
A-Si(R')₃ (3)
formules (1) à (3) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en C₁-C₈, ou phényle,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (4) suivante : 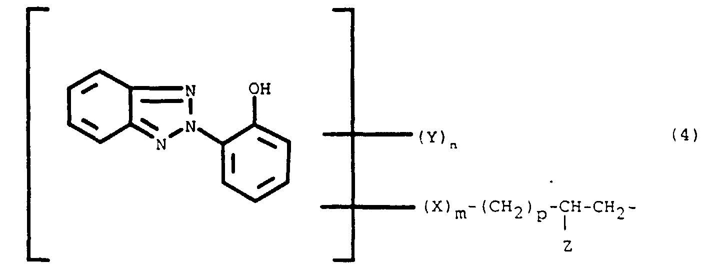 formule (4) dans laquelle :
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1, étant entendu que lorsque m est nul, ou lorsque m est égal à 1 et que X représente NH, alors nécéssairement (i) n ne peut être nul et (ii) l'un au moins des Y est un radical alcoxy,
- p représente un nombre entier compris entre 1 et 10, inclusivement.

2. Composés selon la revendication 1, répondant à la formule (1) ou à la formule (2), caractérisés par le fait que les radicaux R sont des radicaux alkyle.

3. Composés selon la revendication 2, caractérisés par le fait que les radicaux R sont des radicaux méthyle, éthyle, propyle, n-butyle, n-octyle ou éthyl-2 hexyle.

4. Composés selon la revendication 3, caractérisés par le fait que les radicaux R sont des radicaux méthyle.

5. Composés selon l'une quelconque des revendications précédentes, répondant à la formule (1), caractérisés par le fait que les radicaux B sont des radicaux alkyle.

6. Composés selon la revendication 5, caractérisés par le fait que les radicaux B sont des radicaux méthyle, éthyle, propyle, n-butyle, n-octyle ou éthyl-2 hexyle.

7. Composés selon la revendication 6, caractérisés par le fait que les radicaux B sont radicaux méthyle.

8. Composés selon l'une quelconque des revendications précédentes, répondant à la formule (1), caractérisés par le fait que r est compris entre 0 et 3 inclusivement, et s est compris entre 0 et 3 inclusivement.

9. Composés selon l'une quelconque des revendications 1 à 4, répondant à la formule (2), caractérisés par le fait que t + u est compris entre 3 et 5 inclusivement.

10. Composés selon la revendication 1, répondant à la formule (3), caractérisés par le fait que les radicaux R' sont des radicaux alkyle choisis parmi les radicaux méthyle, éthyle, propyle, n-butyle, n-octyle ou éthyl-2 hexyle.

11. Composés selon la revendication 10, caractérisés par le fait que les radicaux R' sont des radicaux méthyle.

12. Composés selon l'une quelconque des revendications précédentes, caractérisés par le fait que n est non nul, de préférence égal à 1 ou 2, et Y est choisi parmi méthyle, ter.-butyle et alcoxy en C₁-C₄.

13. Composés selon la revendication 12, caractérisés par le fait que Y est méthyle ou méthoxy.

14. Composés selon la revendication 13, caractérisés par le fait que Y est méthoxy.

15. Composés selon l'une quelconque des revendications précédentes, caractérisés par le fait que Z est hydrogène ou méthyle.

16. Composés selon l'une quelconque des revendications précédentes, caractérisés par le fait que m est non nul et que X représente O.

17. Composés selon l'une quelconque des revendications précédentes, caractérisés par le fait que p est égal à 1.

18. Composés selon l'une quelconque des revendications précédentes, caractérisés par le fait que l'accrochage du chainon -(X)ₘ-(CH₂)ₚ-CH(Z)-CH₂- sur le motif benzotriazole se fait en position 3, 4, 4' ou 5 de ce dernier.

19. Composés selon la revendication 18, caractérisés par le fait que ledit accrochage se fait en position 3, 4 ou 5.

20. Composés selon l'une quelconque des revendications précédentes, caractérisés par le fait que l'accrochage du substituant Y sur le motif benzotriazole se fait en position(s) 3, 4, 4', 5 et/ou 6 de ce dernier.

21. Dérivés de benzotriazole à insaturation éthylénique répondant à la formule : 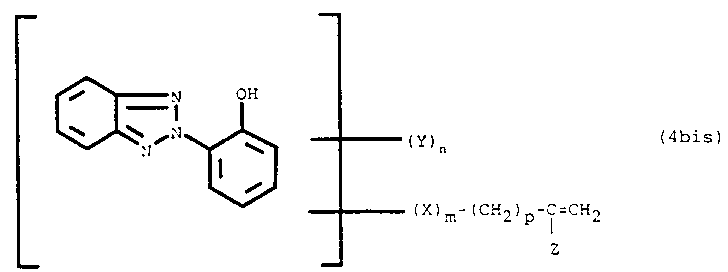 dans laquelle Y, X, Z, n, m et p ont la signification donnée à la revendication 1.

22. Dérivés selon la revendication 21, caractérisés par le fait que n est non nul, de préférence égal à 1 ou 2, et Y est choisi parmi méthyle, ter.-butyle et alcoxy en C₁-C₄.

23. Dérivés selon la revendication 22, caractérisés par le fait que Y est méthyle ou méthoxy.

24. Dérivés selon la revendication 23, caractérisés par le fait que Y est méthoxy.

25. Dérivés selon l'une quelconque des revendications 21 à 24, caractérisés par le fait que Z est hydrogène ou méthyle.

26. Dérivés selon l'une quelconque des revendications 21 à 25, caractérisés par le fait que m est non nul et que X représente O.

27. Dérivés selon l'une quelconque des revendications 21 à 26, caractérisés par le fait que p est égal à 1.

28. Dérivés selon l'une quelconque des revendications 21 à 27, caractérisés par le fait que l'accrochage du chainon -(X)ₘ-(CH₂)ₚ-C(Z)=CH₂ sur le motif benzotriazole se fait en position 3, 4, 4' ou 5 de ce dernier.

29. Dérivés selon la revendication 28, caractérisés par le fait que ledit accrochage se fait en position 3, 4 ou 5.

30. Dérivés selon l'une quelconque des revendications 21 à 29, caractérisés par le fait que l'accrochage du substituant Y sur le motif benzotriazole se fait en position(s) 3, 4, 4', 5 et/ou 6 de ce dernier.

31. Utilisation non therapeutique des composés de formule (1)-(3) ou des dérivés de formule (4bis) tels que définis à l'une quelconque des revendications, respectivement, 1 à 20 et 21 à 30, à titre de filtres solaires actifs dans le domaine des UV-A et/ou UV-B.

32. Composition cosmétique, en particulier pour la filtration des rayons ultraviolets, caractérisée par le fait qu'elle comprend, dans un support cosmétiquement acceptable, une quantité efficace d'au moins un des composés définis à l'une quelconque des revendications 1 à 20.

33. Composition cosmétique selon la revendication 32, caractérisée par le fait que ledit support cosmétiquement acceptable contient au moins une phase grasse ou un solvant organique.

34. Composition cosmétique selon la revendication 33, caractérisée par le fait que ledit support se présente sous la forme d'une émulsion de type huile-dans-eau ou eau-dans-huile, de préférence huile-dans-eau.

35. Composition cosmétique selon l'une des revendications 32 à 34, caractérisée par le fait que la teneur en composé(s) filtrant(s) est comprise entre 0,1 et 20 % en poids par rapport au poids total de la composition.

36. Composition cosmétique selon la revendication 35, caractérisée par le fait que ladite teneur est comprise entre entre 0,5 et 10 % en poids.

37. Procédé de protection non-therapeutique de la peau et/ou des cheveux contre le rayonnement ultraviolet, en particulier le rayonnement solaire, caractérisé par le fait qu'il consiste à appliquer sur la peau et/ou les cheveux une quantité efficace d'au moins un composé, ou d'au moins un dérivé ou d'au moins une composition tels que définis à l'une quelconque des revendications précédentes.

## Claims

1. Compounds of formulae: 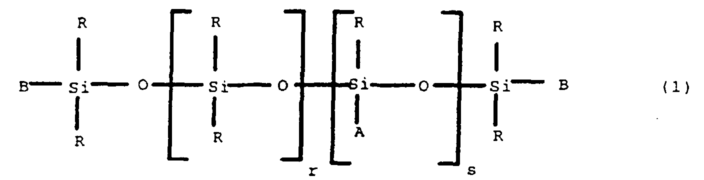 or 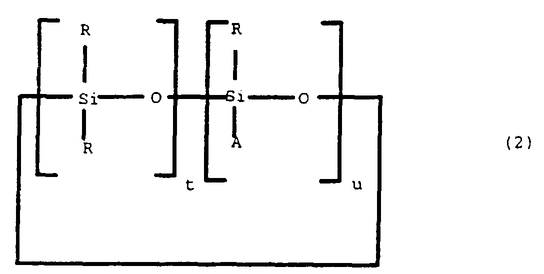 or
A-Si(R')₃ (3)
in which formulae (1) to (3):
- R, which may be identical or different, are chosen from C₁-C₁₀ alkyl, phenyl and 3,3,3-trifluoropropyl radicals, at least 80 % in numerical terms of the radicals R being methyl,
- B, which may be identical or different, are chosen from the above radicals R and the radical A defined below,
- R', which may be identical or different, are chosen from C₁-C₈ alkyl radicals and phenyl radicals,
- r is an integer between 0 and 50 inclusive, and s is an integer between 0 and 20 inclusive, on condition that, if s is zero, then at least one of the two symbols B denotes A,
- u is an integer between 1 and 6 inclusive, and t is an integer between 0 and 10 inclusive, on the understanding that t + u is equal to or greater than 3,
- and the symbol A denotes a univalent radical linked directly to a silicon atom, and which corresponds to the following formula (4): 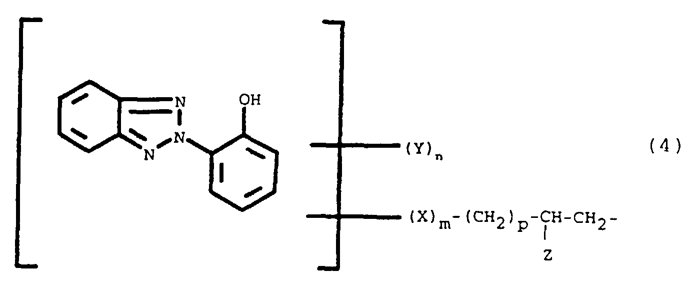
- Y, which may be identical or different, are chosen from C₁-C₈ alkyl radicals, halogens and C₁-C₄ alkoxy radicals, on the understanding that, in the last case, two adjacent radicals Y on the same aromatic ring can form together an alkylidenedioxy group in which the alkylidene group contains from 1 to 2 carbon atoms,
- X represents O or NH,
- Z represents hydrogen or a C₁-C₄ alkyl radical,
- n is an integer between 0 and 3 inclusive,
- m is 0 or 1, on the understanding that, when m is zero, or when m is equal to 1 and X represents NH, then necessarily (i) n cannot be zero and (ii) at least one of the radicals Y is an alkoxy radical,
- p represents an integer between 1 and 10 inclusive.

2. Compounds according to Claim 1, corresponding to the formula (1) or to the formula (2), characterized in that the radicals R are alkyl radicals.

3. Compounds according to Claim 2, characterized in that the radicals R are methyl, ethyl, propyl, n-butyl, n-octyl or 2-ethylhexyl radicals.

4. Compounds according to Claim 3, characterized in that the radicals R are methyl radicals.

5. Compounds according to any one of the preceding claims, corresponding to the formula (1), characterized in that the radicals B are alkyl radicals.

6. Compounds according to Claim 5, characterized in that the radicals B are methyl, ethyl, propyl, n-butyl, n-octyl or 2-ethylhexyl radicals.

7. Compounds according to Claim 6, characterized in that the radicals B are methyl radicals.

8. Compounds according to any one of the preceding claims, corresponding to the formula (1), characterized in that r is between 0 and 3 inclusive and s is between 0 and 3 inclusive.

9. Compounds according to any one of Claims 1 to 4, corresponding to the formula (2), characterized in that t + u is between 3 and 5 inclusive.

10. Compounds according to Claim 1, corresponding to the formula (3), characterized in that the radicals R' are alkyl radicals chosen from methyl, ethyl, propyl, n-butyl, n-octyl and 2-ethylhexyl radicals.

11. Compounds according to Claim 10, characterized in that the radicals R' are methyl radicals.

12. Compounds according to any one of the preceding claims, characterized in that n is not zero and preferably equal to 1 or 2, and Y is chosen from methyl, tert-butyl and C₁-C₄ alkoxy.

13. Compounds according to Claim 12, characterized in that Y is methyl or methoxy.

14. Compounds according to Claim 13, characterized in that Y is methoxy.

15. Compounds according to any one of the preceding claims, characterized in that Z is hydrogen or methyl.

16. Compounds according to any one of the preceding claims, characterized in that m is not zero and in that X represents O.

17. Compounds according to any one of the preceding claims, characterized in that p is equal to 1.

18. Compounds according to any one of the preceding claims, characterized in that the coupling of the chain link -(X)ₘ-(CH₂)ₚ-CH(Z)-CH₂- to the benzotriazole unit takes place at position 3, 4, 4' or 5 of the latter.

19. Compounds according to Claim 18, characterized in that the said coupling takes place at position 3, 4 or 5.

20. Compounds according to any one of the preceding claims, characterized in that the coupling of the substituent Y to the benzotriazole unit takes place at position(s) 3, 4, 4', 5 and/or 6 of the latter.

21. Ethylenically unsaturated benzotriazole derivatives corresponding to the formula: 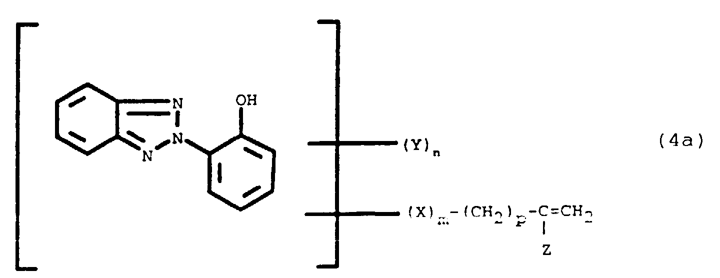 in which Y, X, Z, n, m and p have the meaning given in Claim 1.

22. Derivatives according to Claim 21, characterized in that n is not zero and is preferably equal to 1 or 2, and Y is chosen from methyl, tert-butyl and C₁-C₄ alkoxy.

23. Derivatives according to Claim 22, characterized in that Y is methyl or methoxy.

24. Derivatives according to Claim 23, characterized in that Y is methoxy.

25. Derivatives according to any one of Claims 21 to 24, characterized in that Z is hydrogen or methyl.

26. Derivatives according to any one of Claims 21 to 25, characterized in that m is not zero and in that X represents O.

27. Derivatives according to any one of Claims 21 to 26, characterized in that p is equal to 1.

28. Derivatives according to any one of Claims 21 to 27, characterized in that the coupling of the chain link -(X)ₘ-(CH₂)ₚ-C(Z)=CH₂ to the benzotriazole unit takes place at position 3, 4, 4' or 5 of the latter.

29. Derivatives according to Claim 28, characterized in that the said coupling takes place at position 3, 4 or 5.

30. Derivatives according to any one of Claims 21 to 29, characterized in that the coupling of the substituent Y to the benzotriazole unit takes place at position(s) 3, 4, 4', 5 and/or 6 of the latter.

31. Non-therapeutic use of the compounds of formulae (1)-(3) or of the derivatives of formula (4a) as are defined in any one of Claims 1 to 20 and 21 to 30, respectively, as sunscreen agents which are active in the UV-A and/or UV-B range.

32. Cosmetic composition, especially for screening out ultraviolet rays, characterized in that it comprises, in a cosmetically acceptable vehicle, an effective amount of at least one of the compounds defined in any one of Claims 1 to 20.

33. Cosmetic composition according to Claim 32, characterized in that the said cosmetically acceptable vehicle contains at least one fatty phase or one organic solvent.

34. Cosmetic composition according to Claim 33, characterized in that the said vehicle takes the form of an oil-in-water or water-in-oil type emulsion, preferably oil-in-water.

35. Cosmetic composition according to one of Claims 32 to 34, characterized in that the content of screening compound(s) is between 0.1 and 20 % by weight relative to the total weight of the composition.

36. Cosmetic composition according to Claim 35, characterized in that the said content is between 0.5 and 10 % by weight.

37. Non-therapeutic process for protecting the skin and/or hair against ultraviolet radiation, especially solar radiation, characterized in that it consists in applying to the skin and/or hair an effective amount of at least one compound or of at least one derivative or of at least one composition as are defined in any one of the preceding claims.

## Patentansprüche

1. Verbindungen der Formel: 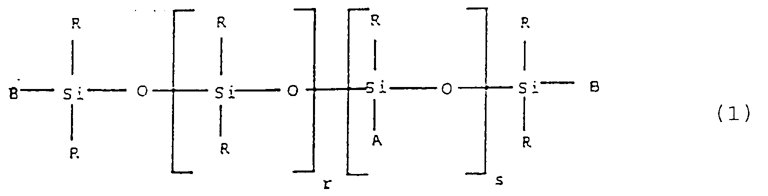 oder 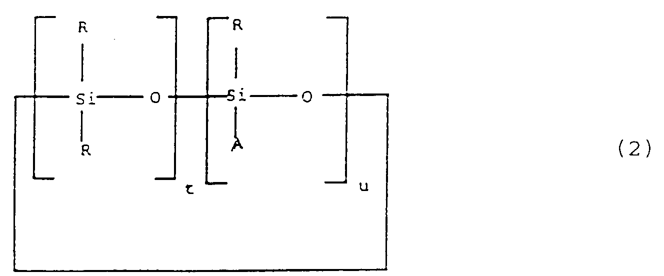 oder
A-Si(R')₃,
wobei in den Formeln (1) bis (3):
- die Gruppen R, die identisch oder voneinander verschieden sind, unter den C₁₋₁₀-Alkylgruppen, Phenyl und 3,3,3-Trifluorpropyl ausgewählt sind, wobei mindestens 80 % der Anzahl der Gruppen R Methyl bedeutet,
- die Gruppen B, die identisch oder voneinander verschieden sind, unter den oben genannten Gruppen R und der nachfolgend definierten Gruppe A ausgewählt sind,
- die Gruppen R', die identisch oder voneinander verschieden sind, unter den C₁₋₈-Alkylgruppen oder Phenyl ausgewählt sind,
- r eine ganze Zahl im Bereich von Null bis einschließlich 50 und s eine ganze Zahl im Bereich von Null bis einschließlich 20 bedeutet, mit der Maßgabe, daß mindestens eine der beiden Gruppen B A bedeutet, wenn s Null ist,
- u eine ganze Zahl im Bereich von 1 bis einschließlich 6 und t eine ganze Zahl im Bereich von Null bis einschließlich 10 bedeutet, mit der Maßgabe, daß t+u mindestens 3 ist, und
- die Gruppe A eine einwertige direkt an ein Siliciumatom gebundene Gruppe bedeutet, die der folgenden Formel (4) entspricht: 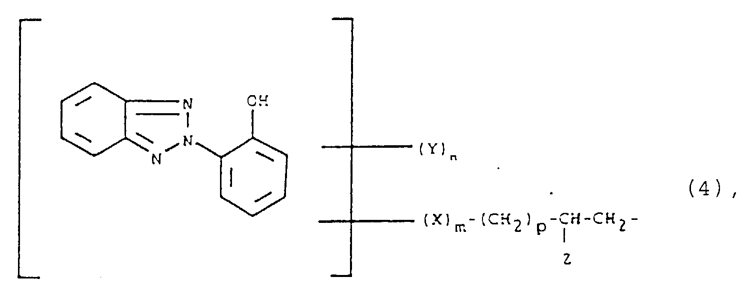 wobei in der Formel (4) bedeuten:
- die Gruppen Y, die identisch oder voneinander verschieden sind, C₁₋₈-Alkyl, Halogen und C₁₋₄-Alkoxy, mit der Maßgabe, daß im letzten Fall zwei aneinandergrenzende Y des gleichen aromatischen Rings gemeinsam eine Alkylidendioxygruppe bilden können, wobei die Alkylidengruppe 1 bis 2 Kohlenstoffatome aufweist,
- X O oder NH,
- Z Wasserstoff oder C₁₋₄-Alkyl,
- n eine ganze Zahl im Bereich von Null bis einschließlich 3,
- m Null oder 1, mit der Maßgabe, daß, falls m Null ist oder m 1 ist und X NH bedeutet, zwangsläufig (i) n nicht Null sein kann und (ii) mindestens eine der Gruppen Y Alkoxy bedeutet,
- p eine ganze Zahl von 1 bis einschließlich 10.

2. Verbindungen nach Anspruch 1, die der Formel (1) oder der Formel (2) entsprechen, dadurch gekennzeichnet, daß die Gruppen R Alkylgruppen sind.

3. Verbindungen nach Anspruch 2, dadurch gekennzeichnet, daß die Gruppen R die Gruppen Methyl, Ethyl, Propyl, n-Butyl, n-Octyl oder 2-Ethylhexyl bedeuten.

4. Verbindungen nach Anspruch 3, dadurch gekennzeichnet, daß die Gruppen R Methyl bedeuten.

5. Verbindungen nach einem der vorhergehenden Ansprüche, die der Formel (1) entsprechen, dadurch gekennzeichnet, daß die Gruppen B Alkylgruppen bedeuten.

6. Verbindungen nach Anspruch 5, dadurch gekennzeichnet, daß die Gruppen B die Gruppen Methyl, Ethyl, Propyl, n-Butyl, n-Octyl oder 2-Ethylhexyl bedeuten.

7. Verbindungen nach Anspruch 6, dadurch gekennzeichnet, daß die Gruppen B Methyl bedeuten.

8. Verbindungen nach einem der vorhergehenden Ansprüche, die der Formel (1) entsprechen, dadurch gekennzeichnet, daß r im Bereich von Null bis einschließlich 3 und s im Bereich von Null bis einschließlich 3 liegt.

9. Verbindungen nach einem der Ansprüche 1 bis 4, die der Formel (2) entsprechen, dadurch gekennzeichnet, daß t+u im Bereich von 3 bis einschließlich 5 liegt.

10. Verbindungen nach Anspruch 1, die der Formel (3) entsprechen, dadurch gekennzeichnet, daß die Gruppen R' Alkylgruppen sind, die unter Methyl, Ethyl, Propyl, n-Butyl, n-Octyl oder 2-Ethylhexyl ausgewählt sind.

11. Verbindungen nach Anspruch 10, dadurch gekennzeichnet, daß die Gruppen R' Methyl bedeuten.

12. Verbindungen nach einem der vorhergehenden Ansprüche, dadurch gekennzeichnet, daß n nicht Null und vorzugsweise 1 oder 2 ist und Y unter Methyl, tert.-Butyl und C₁₋₄-Alkoxy ausgewählt ist.

13. Verbindungen nach Anspruch 12, dadurch gekennzeichnet, daß Y Methyl oder Methoxy ist.

14. Verbindungen nach Anspruch 13, dadurch gekennzeichnet, daß Y Methoxy ist.

15. Verbindungen nach einem der vorhergehenden Ansprüche, dadurch gekennzeichnet, daß Z Wasserstoff oder Methyl bedeutet.

16. Verbindungen nach einem der vorhergehenden Ansprüche, dadurch gekennzeichnet, daß m nicht Null ist und X O bedeutet.

17. Verbindungen nach einem der vorhergehenden Ansprüche, dadurch gekennzeichnet, daß p 1 ist.

18. Verbindungen nach einem der vorhergehenden Ansprüche, dadurch gekennzeichnet, daß die Bindung der Kette -(X)ₘ-(CH₂)ₚ-CH(Z)-CH₂- an die Benzotriazoleinheit in der Stellung 3, 4, 4' oder 5 der Einheit erfolgt.

19. Verbindungen nach Anspruch 18, dadurch gekennzeichnet, daß die Bindung in der Stellung 3, 4 oder 5 erfolgt.

20. Verbindungen nach einem der vorhergehenden Ansprüche, dadurch gekennzeichnet, daß die Bindung des Substituenten Y an der Benzotriazoleinheit in der Stellung oder den Stellungen 3, 4, 4' 5 und/oder 6 der Einheit erfolgt.

21. Ethylenisch ungesättigte Benzotrialzolderivate der Formel: 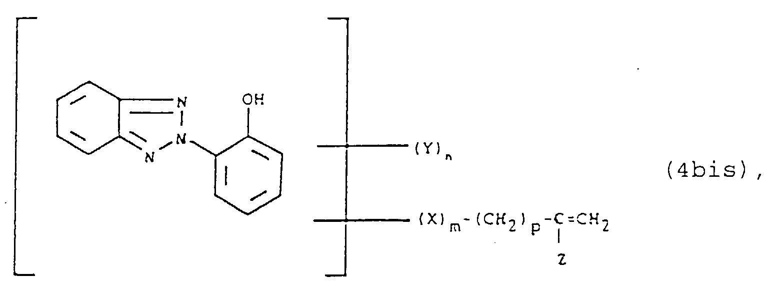 worin Y, X, Z, n, m und p die in Anspruch 1 angegebenen Bedeutungen aufweisen.

22. Derivate nach Anspruch 21, dadurch gekennzeichnet, daß n nicht Null und vorzugsweise 1 oder 2 ist und Y unter Methyl, tert.-Butyl und C₁₋₄-Alkoxy ausgewählt ist.

23. Derivate nach Anspruch 22, dadurch gekennzeichnet, daß Y Methyl oder Methoxy ist.

24. Derivate nach Anspruch 23, dadurch gekennzeichnet, daß Y Methoxy ist.

25. Derivate nach einem der Ansprüche 21 bis 24, dadurch gekennzeichnet, daß Z Wasserstoff oder Methyl bedeutet.

26. Derivate nach einem der Ansprüche 21 bis 25, dadurch gekennzeichnet, daß m nicht Null ist und X O bedeutet.

27. Derivate nach einem der Ansprüche 21 bis 26, dadurch gekennzeichnet, daß p 1 ist.

28. Derivate nach einem der Ansprüche 21 bis 27, dadurch gekennzeichnet, daß die Bindung der Kette -(X)ₘ-(CH₂)ₚ-C(Z)=CH₂ an die Benzotriazoleinheit in der Stellung 3, 4, 4' oder 5 der Einheit erfolgt.

29. Derivate nach Anspruch 28, dadurch gekennzeichnet, daß die Bindung in der Stellung 3, 4 oder 5 erfolgt.

30. Derivate nach einem der Ansprüche 21 bis 29, dadurch gekennzeichnet, daß die Bindung des Substituenten Y an die Benzotriazoleinheit in der Stellung oder den Stellungen 3, 4, 4', 5 und/oder 6 der Einheit erfolgt.

31. Nichttherapeutische Verwendung der Verbindungen der Formeln (1) bis (3) oder der Derivate der Formel (4bis) nach einem der Ansprüche 1 bis 20 bzw. 21 bis 30 als Sonnenschutzfilter, die im UV-A- und/oder UV-B-Bereich wirksam sind.

32. Kosmetische Zusammensetzung, insbesondere um UV-Strahlung zu filtern, dadurch gekennzeichnet, daß sie in einem kosmetisch akzeptablen Träger eine wirksame Menge mindestens einer der Verbindungen nach einem der Ansprüche 1 bis 20 enthält.

33. Kosmetische Zusammensetzung nach Anspruch 32, dadurch gekennzeichnet, daß der kosmetisch akzeptable Träger mindestens eine Fettphase oder ein organisches Lösungsmittel enthält.

34. Kosmetische Zusammensetzung nach Anspruch 33, dadurch gekennzeichnet, daß der Träger in Form einer Öl-in-Wasser-Emulsion oder Wasser-in-Öl-Emulsion und vorzugsweise als Öl-in-Wasser-Emulsion vorliegt.

35. Kosmetische Zusammensetzung nach einem der Ansprüche 32 bis 34, dadurch gekennzeichnet, daß der Gehalt an Filterverbindung oder Filterverbindungen im Bereich von 0,1 bis 20 Gew.-%, bezogen auf das Gesamtgewicht der Zusammensetzung, liegt.

36. Kosmetische Zusammensetzung nach Anspruch 35, dadurch gekennzeichnet, daß der Gehalt im Bereich von 0,5 bis 10 Gew.-% liegt.

37. Nichttherapeutisches Verfahren zum Schutz der Haut und/ oder der Haare gegen UV-Strahlung und insbesondere gegen Sonnenlicht, dadurch gekennzeichnet, daß es darin besteht, auf die Haut oder die Haare eine wirksame Menge mindestens einer Verbindung oder mindestens eines Derivats oder mindestens einer Zusammensetzung nach einem der vorhergehenden Ansprüche aufzutragen.
